# EUROPEAN PATENT APPLICATION

(11) **EP 1 595 497 A1**
(43) Date of publication of application: **16.11.2005**
(21) Application number: 04076344.3
(22) Date of filing: 05.05.2004
(51) Int. Cl.: A61B 5/103, A61B 5/00

(54) **Terminal device and wireless data transmission network**

(71) Applicant: Drakeley Consulting Llc, Houston, Texas 77002 (US)
(72) Inventor: Crescini, Matteo, 20034 Giussano MI (IT)

(57) **Abstract**

A terminal device (10) for measuring at least one physiological parameter on a subject and radiotransmitting the value of the measured parameter to a central processor, comprising a housing including therein
- an electronic control card (11) comprising a radio frequency transceiver (1) and an analogue/digital converter (2) connected thereto,
- an antenna (16) connected to the transceiver (1),
- an acceleration sensor (12) connected to the electronic card (11),
- an on/off switch (15) connected to the electronic card (11), and
- a connector (13), connected to the electronic card (11), suitable to be connected to at least one external sensor of a physiological parameter.

## Description

The present invention concerns a terminal device and a wireless data transmission network comprising such a device.

The present invention also concerns a use of the terminal device and of the network for measuring physiological parameters and radiotransmitting the measured values to a central processor.

The present invention also concerns a use of the terminal device and of the network in a field selected from the group comprising the medical field, the health field, the sanitary field, the agricultural field and the zootechnic field.

There are structures like, for example, hospitals, health centres, old people's homes in which it is necessary to measure some parameters such as, for example, body temperature, blood pressure, heart beat on a plurality of subjects.

Typically, such parameters must be measured many times per day or continuously through one day or over many days. There is therefore the need for measuring devices that are portable, ergonomic, strong, light, miniaturised and pleasant to the touch with the skin of the user so that he/she can easily deal with them, even for a prolonged time period.

Moreover, the parameters measured on the various subjects must then be collected and sent to a central computer for processing by the operators involved. To do this it is advantageous to use a plurality of terminal measurement devices suitably connected together and to the central computer through a data transmission network.

The use of a cabled network is, however, very disadvantageous in terms of costs and difficulty of installation especially in cases of installation in already existing structures. There is therefore the need for a low cost data transmission network that can be easily installed also in already existing structures.

The present invention thus relates, in a first aspect thereof, to a terminal device for measuring at least one physiological parameter on a subject and radiotransmitting the value of the measured parameter to a central processor, the terminal device comprising a housing including therein
- an electronic control card comprising a radio frequency transceiver and an analogue/digital converter connected thereto,
- an antenna connected to the transceiver,
- an acceleration sensor connected to the electronic card,
- an on/off switch connected to the electronic card, and
- a connector, connected to the electronic card, suitable to be connected to at least one external sensor of a physiological parameter.
   In the present description and claims, the expression "physiological parameter" is used to indicate any parameter relating to a human, animal or vegetable subject.
   The dependent claims relate to particular embodiments of the invention.
   Advantageously, the analogue/digital converter is suitable to convert an analogue signal coming from the acceleration sensor and/or the external sensor into a corresponding digital signal and supply it to the transceiver for its radio frequency transmission through the antenna.
   Typically, the acceleration sensor has at least two outputs for providing two signals representatives of the acceleration measured along two reference perpendicular axes. Typically, the acceleration sensor has two analogue outputs and/or two digital outputs for such signals.
   Advantageously, the housing also comprises a battery for power supplying the terminal device. Advantageously, the battery is also suitable to power supply said at least one external sensor through the connector.
   Advantageously, the connector comprises at least one output for power supplying, through the battery, said at least one external sensor.
   Advantageously, the connector is adapted to be connected to at least one external sensor of physiological parameter selected from the group comprising body temperature sensors, pH meters, acidity meters, relative humidity (RH) meters, biopotential meters, meters of ionic concentrations in solution, flow meters, blood pressure meters and heart beat meters, breathing rate meters, electro-cardiographic (ECG) meters, orthostasis meters, blood-oxygenation meters, latent tremor meters and electrical conductivity meters.
   Advantageously, the connector comprises at least one input suitable to be connected to said at least one external sensor of a physiological parameter.
   Advantageously, said at least one input of the connector is suitable to receive an analogue signal from said at least one external sensor.
   Advantageously, said at least one input of the connector is suitable to receive a digital signal from said at least one external sensor.
   Typically, the electronic card comprises two analogue inputs connected to the acceleration sensor. Said two analogue inputs are advantageously connected to the analogue/digital converter.
   The electronic card may comprise two digital inputs connected to the acceleration sensor. Said two digital inputs are advantageously directly connected to the transceiver.
   Typically, the electronic card also comprises at least one further analogue input connected to the connector. Said at least one further analogue input is advantageously connected to the analogue/digital converter.
   Typically, the electronic card also comprises four digital inputs connected to the connector. The electronic card also comprises a buffer connected between the four digital inputs and the transceiver.
   Advantageously, the terminal device also comprises a display for displaying the value of the physiological parameter measured by the external sensor and the movement information detected by the acceleration sensor.
   Advantageously, the connector is connected to at least one physiological parameter sensor.
   The present invention relates, in a second aspect thereof, to a use of a terminal device according to the first aspect of the invention for measuring at least one physiological parameter on a subject, through connection to at least one suitable sensor, and radiotransmitting the value of the measured parameter to a central processor.
   Typically, said physiological parameter is selected from the group comprising body temperature, pH, acidity, relative humidity (RH), biopotential, ionic concentration in solution, flow, blood pressure, heart beat, breathing rate, electrocardiogram, orthostasis, blood oxygenation and latent tremor.
   In a third aspect thereof, the present invention relates the use of a terminal device according to the first aspect of the invention in a field selected from the group comprising the medical field, the health field, the sanitary field, the agricultural field and the zootechnic field.
   In a fourth aspect thereof, the present invention relates to a radiotransmission network for measuring a plurality of physiological parameters on a plurality of subjects and radiotransmitting the measured values to a central processor, said network comprising
- a plurality of terminal devices according to the first aspect of the invention for measuring each at least one physiological parameter on a subject and radiotransmitting the value of the measured parameter;
- a plurality of routers, each connected to at least one respective terminal device, to radioreceive from it the value of said at least one measured parameter and radiotransmit it along the network;
- a gateway suitable to radioreceive from the plurality of routers the values of the physiological parameters measured by the respective terminal devices and to supply them to the central processor.
   As to the structural and functional features of the terminal devices reference is made to what disclosed above with reference to the first aspect of the invention.
   In a fifth aspect thereof, the present invention relates to a use of a radiotransmission network according to the fourth aspect of the invention for measuring a plurality of physiological parameters on a plurality of subjects and radiotransmitting the measured values to a central processor.
   In a further aspect thereof, the present invention relates to a use of a radiotransmission network according to the fourth aspect of the invention in a field selected from the group comprising the medical field, the health field, the sanitary field, the agricultural field and the zootechnic field.
   Further characteristics and advantages of the present invention shall become clearer from the following detailed description of a preferred embodiment thereof, made with reference to the attached drawings. In such drawings,
- figure 1 is a schematic representation of a terminal device according to the invention;
- figure 2 is a schematic representation of a radiotransmission network according to the invention.

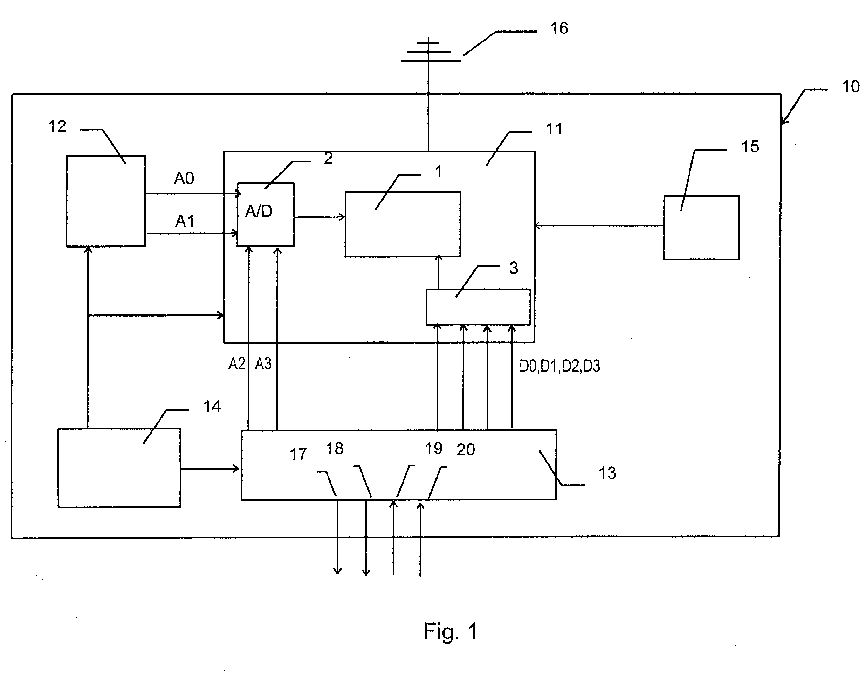

Figure 1 schematically shows a preferred embodiment of a terminal device 10 according to the invention comprising a housing (not shown) including therein an electronic control card 11, an acceleration sensor 12, a connector 13, a battery 14, an on/off switch 15 and an antenna 16.

In turn, the electronic control card 11 comprises an analogue/digital converter 2, a radio frequency transceiver 1 and, typically, also a buffer 3.

The acceleration sensor 12 is typically a conventional device suitable to measure the acceleration along two perpendicular axes and having two outputs for providing the values measured along such axes. The two outputs of the acceleration sensor 12 are connected to the electronic card 11.

Advantageously, the acceleration sensor 12 is a low power consumption device like, for example, the models ADXL 202 or ADXL 213 of the company Analog Devices, Inc. (MA, USA).

For example, the model ADXL 202 has both two analogue outputs and two digital outputs. In the case in which the two analogue outputs are used, the signals provided by such outputs are converted by the analogue/digital converter 2, before being sent to the transceiver 1 for radio frequency transmission through the antenna 16.

The acceleration sensor 12 is used in the cases in which it is necessary to detect movement information. For example, in the case of a patient in a hospital or an old people's home having Parkinson's disease, the terminal device 10 of the invention can be applied onto the patient to measure his/her speed of movement when he/she walks. The speed of movement is, indeed, indicative of the state of progression of Parkinson's disease in the patient.

The battery 14 is used to feed the electronic card 11, the acceleration sensor 12 and to supply power to an external sensor through the connector 13. Advantageously, it is a battery of the conventional type with low energy consumption and small in size.

The connector 13 is suitable to connect the terminal device 10 to one or more external sensors of physiological parameters. In the illustrated embodiment, it is suitable to connect the terminal device 10 to two analogue or digital external sensors.

For example, the connector 13 is a self-locking circular miniaturised device with 4 poles available from the company LEMO S.A. (Switzerland). In particular, such a device has 4 lines 17, 18, 19, 20 of which two can be used for the connection to two external sensors with analogue output and two for providing such sensors with power supply and ground. Such a connector can also be configured as a serial port for the connection to two external sensors having a serial output.

Typical examples of sensors to which the connector 13 can be connected are body temperature sensors, pH meters, acidity meters, relative humidity (RH) meters, biopotential meters, meters of ionic concentrations in solution, flow meters, blood pressure meters and heart beat meters, breathing rate meters, electro-cardiographic (ECG) meters, orthostasis meters, blood-oxygenation meters, latent tremor meters and electrical conductivity meters.

The electronic card 11 comprises two analogue (or digital) inputs A0, A1 for receiving the analogue (or digital) signals from the acceleration sensor 12, two analogue inputs A2, A3 for receiving analogue signals coming from analogue external sensors and four digital inputs D0, D1, D2, D3 for receiving digital signals coming from digital external sensors having serial output.

The analogue/digital converter 2 is suitable to convert the analogue signals coming from the inputs A2, A3 into corresponding digital signals and for providing them to the transceiver 1 for their radiotransmission through the antenna 16.

The buffer 3 is connected to the four digital inputs D0, D1, D2, D3 to temporarily memorise the digital data received by the external sensor with serial output, before sending them to the transceiver 1. The latter is suitable to radiotransmit the data coming from the buffer 3 in packets.

The transceiver 1 is typically suitable to make a serial communication, for example of the RS-232, Ethernet, 802.11 type. For example, it is a conventional universal asynchronous receiver-transmitter (UART).

The electronic card 11 is, for example, a conventional miniaturised low power consumption device of the type available from the company Millenial Net Inc. (MA, USA), model i-Bean®.

The switch 15 activates and deactivates the operation of the terminal device 10.

Advantageously, the terminal device 10 also externally comprises a display (not shown) to display the value of the physiological parameter measured by the external sensor and/or the movement information detected by the acceleration sensor 12 to a user.

The terminal device 10 of the invention, comprising devices that can be miniaturised, can be made portable, light and small in size (for example, of 35.5mm X 35.5mm X 16mm). Moreover, the terminal device 10 - being able to be easily inserted in a casing in the shape, for example, of a small band, wristband, watch or ankle support - can be made ergonomic and pleasant to the touch with the skin of a user. In this way, the terminal device 10 of the invention can easily be applied to the user and worn by him/her without effort and hindrance even for long time periods.

Moreover, the terminal device 10 of the invention, comprising low power consumption components, involves low operating costs and can be battery operated. This allows the user to move freely during the measurement of the physiological parameters and/or the detection of movement information.

The terminal device 10 of the invention can advantageously be used to measure one or more physiological parameters on a human, animal or vegetable subject.

Terminal devices 10 of the invention can advantageously be used in a radiotransmission network to measure a plurality of physiological parameters on a plurality of subjects and radiotransmit them to a central processor.

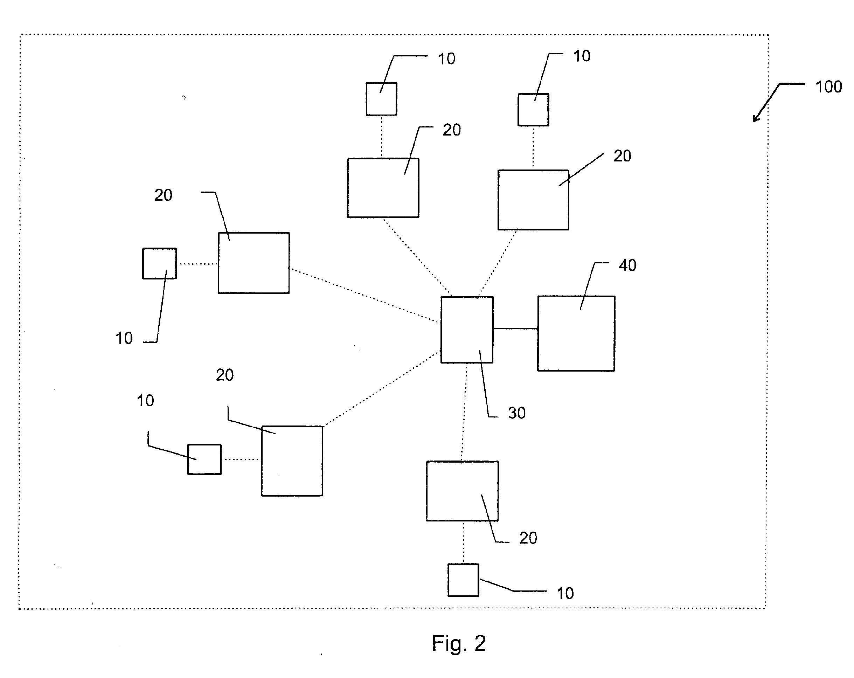

Figure 2 shows a radiotransmission network 100 according to the invention comprising a plurality of terminal devices 10 according to the invention, a plurality of routers 20 and a gateway 30 connected to a central processor 40.

In the embodiment shown, the routers 20 are each associated with a respective terminal device 10, to radioreceive from it the value or values of the physiological parameters measured on a subject and radiotransmit them along the network 100 towards the gateway 30. According to other embodiments not shown, each router can also be associated with more than one terminal device. Moreover, one or more terminal device(s) can radiotransmit the value(s) of the measured physiological parameter(s) directly to the gateway.

The routers 20 are connected to the gateway 30 through suitable conventional radio links.

Typically, the data transmission along the network 100 between the terminal devices 10, the routers 20 and the gateway 30 takes place in radio frequency, according to techniques known in the art.

The gateway 30 is suitable to radioreceive from the plurality of routers 20 the values of the parameters measured by the respective terminal devices 10 and supplying them to the central processor 40, according to conventional techniques.

The routers 20 and the gateway 30 are conventional devices. For example, they are available from the company Millenial Net Inc., (MA, USA), model i-Bean® 510.

The central processor 40 is typically a conventional personal computer equipped with a suitable software for processing the data received from the gateway 30.

The network 100 of the invention, being a wireless transmission network, can easily be installed at a low cost, also in already existing structures.

For example, the network 100 can be used in a hospital, an emergency ward, a local health corporation, a general surgery, a surgery, a health centre, an old people's home, an integrated day centre, a gym, a sports field, a transport system, such as ambulances, to measure physiological parameters of interest on various subjects of the structure and then send them to the central processor 40 where they can be stored, displayed and processed according to the applications.

For example, each terminal device 10 can be used to measure, on a subject of the structure, through connection to one or more external sensors, one or more physiological parameters of interest such as, for example, body temperature, pH, acidity, relative humidity (RH), biopotential, ionic concentration in solution, flow, blood pressure, heart beat, breathing rate, electrocardiogram, orthostasis, blood oxygenation and latent tremor.

The values measured by each terminal device 10 are then sent through the transceiver 1 and the antenna 16 to the respective router 20 which shall take care of routing the data, according to known technologies, along the network 100 towards the gateway 30 and, therefore, the central processor 40.

Depending on the specific requirements and applications, it shall be necessary to connect the terminal device 10 to the most appropriate external sensor, through the connector 13.

For example, should it be necessary to carry out a measurement of free radicals on a subject in a health centre, such a measurement can be carried out by connecting the terminal device 10 to a heart beat sensor. The heart beat is, indeed, indicative of the presence of free radicals.

The network 100, being wireless, is also adapted to be easily installed outdoors like, for example, in a cultivated field.

For example, the network 100 can be installed in a vineyard. In this case, the terminal devices 10 can be applied to the rows of the vineyard and connected to suitable PH sensors to measure the acidity of the grapes. This allows useful information on the state of progress of maturation of the grapes of the various rows to be always available on the central processor 40 so as to be able to plan the grape harvest times.

Moreover, the network 100 is also adapted to be used in the zootechnic field. For example, it can easily be installed in a stable or an animal farm to measure one or more physiological parameters of interest on the animals of the stable or farm.

## Claims

1. A terminal device (10) for measuring at least one physiological parameter on a subject and radiotransmitting the value of the measured parameter to a central processor, comprising a housing including therein
- an electronic control card (11) comprising a radio frequency transceiver (1) and an analogue/digital converter (2) connected thereto,
- an antenna (16) connected to the transceiver (1),
- an acceleration sensor (12) connected to the electronic card (11),
- an on/off switch (15) connected to the electronic card (11), and
- a connector (13), connected to the electronic card (11), suitable to be connected to at least one external sensor of a physiological parameter.

2. Terminal device (10) according to claim 1, wherein the analogue/digital converter (2) is connected to the acceleration sensor (12) to convert at least one analogue signal coming from the acceleration sensor (12) into a corresponding digital signal and supply it to the transceiver (1) for its transmission through the antenna (16).

3. Terminal device (10) according to claim 1 or 2, wherein the housing also comprises a battery (14) for power supplying the terminal device (10).

4. Terminal device (10) according to claim 3, wherein the connector (13) comprises at least one output (17; 18) for power supplying, through the battery (14), said at least one external sensor.

5. Terminal device (10) according to any one of claims 1 to 4, wherein the connector (13) comprises at least one input (19; 20) suitable to be connected to said at least one external sensor of a physiological parameter.

6. Terminal device (10) according to claim 5, wherein said at least one input (19; 20) of the connector (13) is suitable to receive an analogue signal from said at least one external sensor.

7. Terminal device (10) according to claim 5 or 6, wherein said at least one input (19; 20) of the connector (13) is suitable to receive a digital signal from said at least one external sensor.

8. Terminal device (10) according to any one of claims 1 to 7, wherein the electronic card (11) comprises two analogue inputs (A0, A1) connected to the acceleration sensor (12).

9. Terminal device (10) according to claim 8, wherein said two analogue inputs (A0, A1) are connected to the analogue/digital converter (2).

10. Terminal device (10) according to any one of claims 1 to 9, wherein the electronic card (11) also comprises at least one further analogue input (A2; A3) connected to the connector (13).

11. Terminal device (10) according to claim 10, wherein said at least one further analogue input (A2; A3) is connected to the analogue/digital converter (2).

12. Terminal device (10) according to any one of claims 1 to 11, wherein the electronic card (11) also comprises four digital inputs (D0, D1, D2, D3) connected to the connector (13).

13. Terminal device (10) according to claim 12, wherein the electronic card (11) also comprises a buffer (3) connected between the four digital inputs (D0, D1, D2, D3) and the transceiver (1).

14. Terminal device (10) according to any one of claims 1 to 13, also comprising a display for displaying the value of the physiological parameter measured by the external sensor and the movement information detected by the acceleration sensor (12).

15. Terminal device (10) according to any one of claims 1 to 14, wherein the connector (13) is connected to at least one physiological parameter sensor.

16. Use of a terminal device (10) according to any one of claims 1 to 15 for measuring at least one physiological parameter on a subject, through connection to at least one suitable sensor, and radiotransmitting the value of the measured parameter to a central processor.

17. Use of a terminal device (10) according to any one of claims 1 to 15 in a field selected from the group comprising the medical field, the health field, the sanitary field, the agricultural field and the zootechnic field.

18. Radiotransmission network (100) for measuring a plurality of physiological parameters on a plurality of subjects and radiotransmitting the measured values to a central processor (40), said network (100) comprising
- a plurality of terminal devices (10) according to any of claims 1 to 15 for measuring each at least one physiological parameter on a subject and radiotransmitting the value of the measured parameter;
- a plurality of routers (20), each connected to at least one respective terminal device (10), to radioreceive from it the value of said at least one measured parameter and radiotransmit it along the network (100);
- a gateway (30) suitable to radioreceive from the plurality of routers (20) the values of the physiological parameters measured by the respective terminal devices (10) and to supply them to the central processor (40).

19. Use of a radiotransmission network (100) according to claim 18 for measuring a plurality of physiological parameters on a plurality of subjects and radiotransmitting the measured values to a central processor (40).

20. Use of a radiotransmission network (100) according to claim 18 in a field selected from the group comprising the medical field, the health field, the sanitary field, the agricultural field and the zootechnic field.
